# EUROPEAN PATENT APPLICATION

(11) **EP 4 473 965 A1**
(43) Date of publication of application: **11.12.2024**
(21) Application number: 23749855.5
(22) Date of filing: 03.02.2023
(51) Int. Cl.: A61K 31/4152, A61P 11/00, A61P 21/00, A61P 25/02, A61P 43/00

(54) **MEDICAL AGENT FOR TREATING OR SUPPRESSING PROGRESSION OF AMYOTROPHIC LATERAL SCLEROSIS**

(30) Priority: 03.02.2022 JP 2022015946
(71) Applicant: Mitsubishi Tanabe Pharma Corporation, Osaka-shi, Osaka 541-8505 (JP)
(72) Inventor: TAKAHASHI, Fumihiro, Osaka-shi, Osaka 541-8505 (JP); USHIROGAWA, Yoshiteru, Osaka-shi, Osaka 541-8505 (JP)
(74) Representative: Hoffmann Eitle
(86) International application number: PCT/JP2023/003542
(87) International publication number: WO 2023/149539

(57) **Abstract**

Disclosed is a medical agent for treating or suppressing progression of at least one symptom selected from a group consisting of amyotrophic lateral sclerosis and symptoms resulting from amyotrophic lateral sclerosis in a subject. The medical agent contains 3-methyl-1-phenyl-2-pyrazolin-5-one or a physiologically acceptable salt thereof, or a hydrate or solvate thereof. A blood uric acid level of the subject before administration of the medical agent is 4.2 mg/dL or higher.

## Description

### [Technical Field]

The present application claims priority based on Japanese Patent Application No. 2022-015946, filed on February 3, 2022, the entire content of which is incorporated by reference into this specification.

The present disclosure relates to a medical agent and a method for treating or suppressing progression of at least one symptom selected from a group consisting of amyotrophic lateral sclerosis and symptoms resulting therefrom.

### [Technical Background]

Amyotrophic lateral sclerosis (ALS) is a progressive and fatal neuromuscular disease characterized by degeneration of both upper motor neurons and lower motor neurons in the brain and spinal cord. ALS patients typically have a life expectancy of 3 to 5 years from the onset of the disease, with a 50% survival rate within 30 months from the onset.

Edaravone is a compound with the chemical name 3-methyl-1-phenyl-2-pyrazolin-5-one. Edaravone is a free radical scavenger and is the second drug approved for ALS in the United States.

Non-Patent Document 1 describes that ALS patients showed increased oxidative stress compared to healthy controls of the same age. Further, Non-patent Document1 examines the efficacy of edaravone as a free radical scavenger.

### [Related Art]

### [Non-Patent Document]

[Non-Patent Document 1] Nagase et al., Redox Report, 2016, Vol. 21, No. 3, pp. 104-112

### [Summary of the Invention]

Clinical trials using edaravone injections include two-group phase 3 trials. However, the results of these clinical trials do not indicate characteristics of patients who respond to edaravone, and it is unclear which changes in parameters correlate with the suppression of decline in the revised ALS Functional Rating Scale (ALSFRS-R). Further, Non-Patent Document 1 describes that some of ALS patients treated with edaravone exhibit increased uric acid levels. However, Non-Patent Document 1 also does not clarify the characteristics of patients who respond to edaravone.

The present disclosure identifies characteristics of subjects for treating or suppressing progression of at least one symptom selected from a group consisting of ALS and symptoms resulting therefrom, and relates to a medical agent that exhibits a significant effect on subjects with these characteristics.

The present inventors have found that administering a medical agent containing 3-methyl-1-phenyl-2-pyrazolin-5-one or a physiologically acceptable salt thereof, or a hydrate or solvate thereof, to a subject with a pre-administration blood uric acid level of 4.2 mg/dL or higher can exhibit a significant effect in treating or suppressing progression of at least one symptom selected from a group consisting of ALS and symptoms resulting therefrom. Based on this finding, further diligent studies are conducted and the present disclosure is accomplished.

The disclosure includes the following embodiments:

### [Aspect 1]

A medical agent for treating or suppressing progression of at least one symptom selected from a group consisting of amyotrophic lateral sclerosis and symptoms resulting from amyotrophic lateral sclerosis in a subject, comprising:
3-methyl-1-phenyl-2-pyrazolin-5-one or a physiologically acceptable salt thereof, or a hydrate or solvate thereof, wherein
a blood uric acid level of the subject before administration of the medical agent is 4.2 mg/dL or higher.

### [Aspect 2]

The medical agent according to Aspect 1, wherein the blood uric acid level is 4.5 mg/dL or higher.

### [Aspect 3]

The medical agent according to Aspect 1, wherein the blood uric acid level is 4.8 mg/dL or higher.

### [Aspect 4]

The medical agent according to any one of Aspects 1 to 3, wherein the subject is a subject for whom a difference (V2 - V1) between a blood uric acid level V1 before administration of the medical agent and a blood uric acid level V2 two weeks or more from start of the administration of the medical agent is greater than 0 mg/dL.

### [Aspect 5]

The medical agent according to any one of Aspects 1 to 4, wherein the administration of the medical agent is intermittent administration or daily administration.

### [Aspect 6]

The medical agent according to any one of Aspects 1 to 5, wherein
an administration route of the medical agent is oral administration or gastric administration, and
a dose per administration of the medical agent is 90 mg to 120 mg converted to 3-methyl-1-phenyl-2-pyrazolin-5-one.

### [Aspect 7]

The medical agent according to any one of Aspects 1 to 6, wherein
an administration route of the medical agent is oral administration or gastric administration, and
a dose per administration of the medical agent is 90 mg to 105 mg converted to 3-methyl-1-phenyl-2-pyrazolin-5-one.

### [Aspect 8]

The medical agent according to any one of Aspects 1 to 7, wherein
the administration of the medical agent is daily administration,
an administration route of the medical agent is oral administration or gastric administration, and
the medical agent is administered once a day, and
a dose per administration of the medical agent is 105 mg converted to 3-methyl-1-phenyl-2-pyrazolin-5-one.

### [Aspect 9]

The medical agent according to any one of Aspects 1 to 7, wherein
the administration of the medical agent is intermittent administration, and
the medical agent is administered in a regimen that repeats a 14-day administration period and a 14-day drug holiday period, or, after an initial 14-day administration period and a subsequent initial 14-day drug holiday period, repeats a 10-day administration period within 14 days followed by a 14-day drug holiday period.

### [Aspect 10]

The medical agent according to any one of Aspects 1 to 7, wherein
the administration of the medical agent is intermittent administration, and
the medical agent is administered in a regimen that, after an initial 14-day administration period followed by an initial 14-day drug holiday period, repeats a 10-day administration period within 14 days followed by a 14-day drug holiday period.

### [Aspect 11]

The medical agent according to any one of Aspects 1 to 10, wherein the subject has an amyotrophic lateral sclerosis (ALS) severity of grade 3, 4, or 5.

### [Aspect 12]

The medical agent according to any one of Aspects 1 to 11, wherein the symptoms resulting from amyotrophic lateral sclerosis include decreased respiratory function, speech impairment, dysphagia, and limb motor dysfunction.

### [Aspect A1]

A method for treating or suppressing progression of at least one symptom selected from a group consisting of amyotrophic lateral sclerosis and symptoms resulting from amyotrophic lateral sclerosis in a subject in need of treating or suppressing progressionof the symptom, comprising:
a step of administering 3-methyl-1-phenyl-2-pyrazolin-5-one or a physiologically acceptable salt thereof, or a hydrate or solvate thereof, to the subject, wherein
a blood uric acid level of the subject before the administration is 4.2 mg/dL or higher.

### [Aspect A2]

The method according to claim A1, wherein the blood uric acid level is 4.5 mg/dL or higher.

### [Aspect A3]

The method according to claim A1, wherein the blood uric acid level is 4.8 mg/dL or higher.

### [Aspect A4]

The method according to Aspect A1, wherein the step is a step of continuing to administer 3-methyl-1-phenyl-2-pyrazolin-5-one or a physiologically acceptable salt thereof, or a hydrate or solvate thereof to the subject, in a case where a difference (V2 - V1) between a blood uric acid level V1 before administration and a blood uric acid level V2 two weeks or more from start of the administration is greater than 0 mg/dL.

### [Aspect A5]

The method according to Aspect A1, wherein the administration in the step is intermittent administration or daily administration.

### [Aspect A6]

The method according to Aspect A1, wherein
an administration route of the step is oral administration or gastric administration, and
a dose per administration is 90 mg to 120 mg converted to 3-methyl-1-phenyl-2-pyrazolin-5-one.

### [Aspect A7]

The method according to Aspect A1, wherein
an administration route of the step is oral administration or gastric administration, and
a dose per administration is 90 mg to 105 mg converted to 3-methyl-1-phenyl-2-pyrazolin-5-one.

### [Aspect A8]

The method according to Aspect A1, wherein
the administration of the step is daily administration,
the administration route is oral administration or gastric administration,
the administration is once a day, and
a dose per administration is 105 mg converted to 3-methyl-1-phenyl-2-pyrazolin-5-one.

### [Aspect A9]

The method according to Aspect A1, wherein
the administration in the step is intermittent administration, and
the administration is carried out in a regimen that repeats a 14-day administration period and a 14-day drug holiday period, or, after an initial 14-day administration period and a subsequent initial 14-day drug holiday period, repeats a 10-day administration period within 14 days followed by a 14-day drug holiday period.

### [Aspect A10]

The method according to Aspect A1, wherein
the administration in the step is intermittent administration, and
the administration is carried out in a regimen that, after an initial 14-day administration period followed by an initial 14-day drug holiday period, repeats a 10-day administration period within 14 days followed by a 14-day drug holiday period.

### [Aspect A11]

The method according to Aspect A1, wherein the subject has an amyotrophic lateral sclerosis (ALS) severity of grade 3, 4, or 5.

### [Aspect A12]

The method according to claim A1, wherein the symptoms resulting from amyotrophic lateral sclerosis include decreased respiratory function, speech impairment, dysphagia, and limb motor dysfunction.

### [Aspect A13]

A method for treating or suppressing progression of a symptom of amyotrophic lateral sclerosis in a subject in need of treating or suppressing progression of the symptom, comprising:
a step of administering 3-methyl-1-phenyl-2-pyrazolin-5-one or a physiologically acceptable salt thereof, or a hydrate or solvate thereof, to the subject, wherein
a blood uric acid level of the subject before the administration is 4.2 mg/dL or higher,
the step is a step of continuing to administer 3-methyl-1-phenyl-2-pyrazolin-5-one or a physiologically acceptable salt thereof, or a hydrate or solvate thereof to the subject, in a case where a difference (V2 - V1) between a blood uric acid level V1 before administration and a blood uric acid level V2 two weeks or more from start of the administration is greater than 0 mg/dL, and
the administration in the step is carried out in a regimen that, after an initial 14-day administration period followed by an initial 14-day drug holiday period, repeats a 10-day administration period within 14 days followed by a 14-day drug holiday period.

### [Aspect B1]

A compound that is 3-methyl-1-phenyl-2-pyrazolin-5-one or a physiologically acceptable salt thereof, or a hydrate or solvate thereof, for use in treating or suppressing progression of at least one symptom selected from a group consisting of amyotrophic lateral sclerosis and symptoms resulting from amyotrophic lateral sclerosis in a subject, wherein
a blood uric acid level of the subject before administration of the compound is 4.2 mg/dL or higher.

### [Aspect B2]

The compound according to Aspect B1, wherein the blood uric acid level is 4.5 mg/dL or higher.

### [Aspect B3]

The compound according to Aspect B1, wherein the blood uric acid level is 4.8 mg/dL or higher.

### [Aspect B4]

The compound according to any one of Aspects B1 to B3, wherein the subject is a subject for whom a difference (V2 - V1) between a blood uric acid level V1 before administration of the compound and a blood uric acid level V2 two weeks or more from start of the administration of the compound is greater than 0 mg/dL.

### [Aspect B5]

The compound according to any one of Aspects B1 to B4, wherein the administration of the compound is intermittent administration or daily administration.

### [Aspect B6]

The compound according to any one of Aspects B1 to B5, wherein
an administration route of the compound is oral administration or gastric administration, and
a dose per administration of the compound is 90 mg to 120 mg converted to 3-methyl-1-phenyl-2-pyrazolin-5-one.

### [Aspect B7]

The compound according to any one of Aspects B1 to B6, wherein
an administration route of the compound is oral administration or gastric administration, and
a dose per administration of the compound is 90 mg to 105 mg converted to 3-methyl-1-phenyl-2-pyrazolin-5-one.

### [Aspect B8]

The compound according to any one of Aspects B1 to B7, wherein
the administration of the compound is daily administration,
an administration route of the compound is oral administration or gastric administration, and the compound is administered once a day, and
a dose per administration of the compound is 105 mg converted to 3-methyl-1-phenyl-2-pyrazolin-5-one.

### [Aspect B9]

The compound according to any one of Aspects B1 to B7, wherein
the administration of the compound is intermittent administration, and
the compound is administered in a regimen that repeats a 14-day administration period and a 14-day drug holiday period, or, after an initial 14-day administration period and a subsequent initial 14-day drug holiday period, repeats a 10-day administration period within 14 days followed by a 14-day drug holiday period.

### [Aspect B10]

The compound according to any one of Aspects B1 to B7, wherein
the administration of the compound is intermittent administration, and
the medical agent is administered in a regimen that, after an initial 14-day administration period followed by an initial 14-day drug holiday period, repeats a 10-day administration period within 14 days followed by a 14-day drug holiday period.

### [Aspect B11]

The compound according to any one of Aspects B1 to B10, wherein
the subject has an amyotrophic lateral sclerosis (ALS) severity of grade 3, 4, or 5.

### [Aspect B12]

The compound according to any one of Aspects B1 to B11, wherein the symptoms resulting from amyotrophic lateral sclerosis include decreased respiratory function, speech impairment, dysphagia, and limb motor dysfunction.

### [Aspect C]

Use of 3-methyl-1-phenyl-2-pyrazolin-5-one or a physiologically acceptable salt thereof, or a hydrate or solvate thereof for manufacturing a medical agent for treating or suppressing progression of at least one symptom selected from a group consisting of amyotrophic lateral sclerosis and symptoms resulting from amyotrophic lateral sclerosis in a subject, wherein a blood uric acid level of the subject before administration of the medical agent is 4.2 mg/dL or higher.

### [Effect of Invention]

According to the subject matter of the present disclosure, for a subject having a specific blood uric acid level, an extremely excellent effect is achieved in treating or suppressing progression of at least one symptom selected from a group consisting of ALS and symptoms resulting therefrom.

### [Brief Description of Drawing]

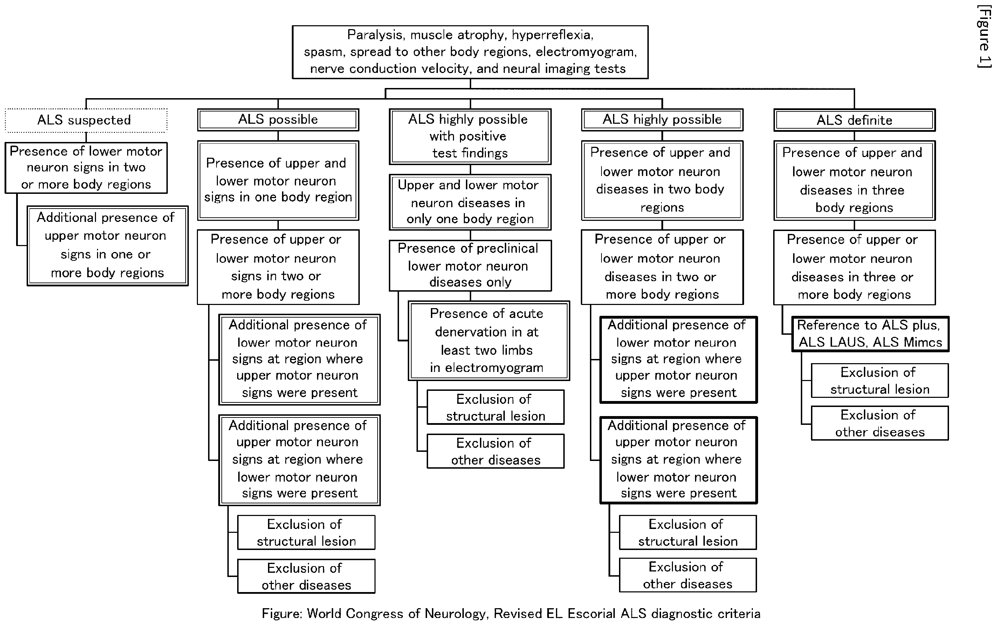

[Fig. 1] Fig. 1 shows the EL Escorial-Revised Airlie House diagnostic criteria.

### [Mode for Carrying Out the Invention]

The entire disclosure of each publication cited in the present specification is incorporated by reference into the present specification. In the present specification, unless otherwise specified, each numerical value can be interpreted by applying a conventional rounding method such as rounding up or down to an appropriate number of significant digits. Further, in the present specification, a notation "X[Z] to Y[Z]" (where X and Y represent any numbers, and [Z] represents any unit attached) means "X[Z] or more and Y[Z] or less" unless otherwise specified. Further, in the present specification, "about" means within a permissible error range for an indicated value, for example, within ±10% (preferably ±5%) of the indicated value.

### 1. Medical agent

The medical agent of the present disclosure contains 3-methyl-1-phenyl-2-pyrazolin-5-one or a physiologically acceptable salt thereof, or a hydrate or solvate thereof (hereinafter may be referred to as an 'active ingredient'). 3-Methyl-1-phenyl-2-pyrazolin-5-one exists in tautomeric forms represented by Formula (1) and Formula (2).

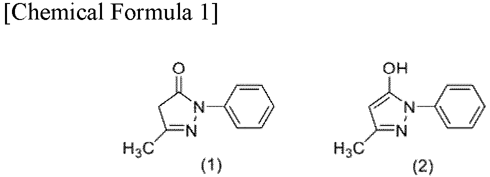

Examples of physiologically acceptable salts of 3-methyl-1-phenyl-2-pyrazolin-5-one include, but are not limited to, salts with mineral acids such as hydrochloric acid, sulfuric acid, hydrobromic acid, and phosphoric acid; salts with organic acids such as methanesulfonic acid, p-toluenesulfonic acid, acetic acid, oxalic acid, citric acid, malic acid, and fumaric acid; salts with alkali metals such as sodium and potassium; salts with alkaline earth metals such as magnesium; ammonium salts; salts with amines such as ethanolamine and 2-amino-2-methyl-1-propanol; and the like.

3-Methyl-1-phenyl-2-pyrazolin-5-one or a physiologically acceptable salt thereof, or a hydrate or solvate thereof, is not particularly limited but can be manufactured, for example, using a method described in European Patent Publication No. 208874 (U.S. Patent Publication No. 4857542 or Japanese Patent Publication No. Hei 5-31523).

A medical agent according to one embodiment of the present disclosure consists only of the active ingredient. In this medical agent, the active ingredient is administered directly to a subject. A medical agent according to another embodiment of the present disclosure contains the active ingredient and a pharmacologically and pharmaceutical acceptable additive. This medical agent can be provided as a formulation in a form commonly known to those skilled in the art.

Examples of the pharmacologically and pharmaceutically acceptable additive include excipients, disintegrants or disintegrating aids, binders, lubricants, coating agents, colorants, diluents, bases, solvents or solubilizing aids, isotonic agents, pH adjustors, stabilizers, propellants, adhesives, dispersants, thickeners, sweeteners, defoaming agents, and the like. These additives may be used individually or in combinations of two or more. Examples of the formulation suitable for oral administration or gastric administration include tablets, capsules, powders, fine granules, granules, liquids, syrups, suspensions, and the like. Examples of the formulation suitable for non-oral administration include injections, intravenous drip infusions, eye drops, patches, suppositories, and the like.

Examples of additives for the formulation suitable for oral administration or gastric administration include: excipients such as glucose, lactose, D-mannitol, starch, and crystalline cellulose; disintegrants or disintegration aids such as carboxymethylcellulose, starch, and calcium carboxymethylcellulose; binders such as hydroxypropylcellulose, hydroxypropylmethylcellulose, polyvinylpyrrolidone, and gelatin; lubricants such as magnesium stearate and talc; coating agents such as hydroxypropylmethylcellulose, white sugar, polyethylene glycol, and titanium oxide; bases such as petrolatum, liquid paraffin, polyethylene glycol, gelatin, kaolin, glycerin, purified water, and hard fat; dispersants such as polyvinyl alcohol and sucrose fatty acid esters; thickeners such as xanthan gum and powdered tragacanth; sweeteners such as sorbitol; stabilizers such as sodium hydrogen sulfite and L-cysteine hydrochloride; pH adjusters such as phosphoric acid, acetic acid, and sodium hydroxide; antifoaming agents such as dimethicone emulsion; solvents such as aqueous solvents like water, buffer solution, saline, and oily solvents like olive oil; fragrances; and the like. These additives can be used individually or in combinations of two or more.

Examples of additives for the formulation suitable for injection or intravenous drip infusion include: dissolving agents or solubilizing aids that can form aqueous or ready-to-use injectables, such as distilled water for injection, physiological saline, and propylene glycol; isotonicity agents, such as glucose, sodium chloride, D-mannitol, and glycerin; pH regulators, such as inorganic acids, organic acids, inorganic bases, and organic bases; and the like. These additives can be used individually or in combinations of two or more.

An injectable formulation containing 3-methyl-1-phenyl-2-pyrazolin-5-one is already in clinical use (generic name: "edaravone"; brand names: "Radicut (registered trademark)," "Radicava (registered trademark)"; manufactured and marketed by Mitsubishi Tanabe Pharma Corporation and the like). Therefore, the above injectable formulation may be used as is as the medical agent of the present disclosure.

The medical agent of the present disclosure can be used for treating or suppressing progression (including halting and delaying the progression) of at least one symptom selected from a group consisting of ALS and symptoms resulting from ALS in a subject. Examples of symptoms resulting from ALS include decreased respiratory function, speech impairment, dysphagia, limb motor dysfunction, and the like. ALS or symptoms resulting therefrom should be interpreted in a broadest sense possible according to technical knowledge in the relevant technical field, and should not be construed in a way that is confined to differences in disease names.

The medical agent of the present disclosure can exhibit a significant effect in treating or suppressing progression of ALS or a symptom resulting therefrom in a subject with specific characteristics, that is, a subject whose blood uric acid level before administration of the medical agent is 4.2 mg/dL or higher.

Among various potential factors that could be related to at least one symptom selected from a group consisting of ALS and symptoms resulting therefrom, such as biomarkers related to oxidative stress (for example, 3-nitrotyrosine), biomarkers related to neuronal apoptosis inhibition (for example, neuronal apoptosis inhibitory proteins such as serum C-reactive protein), biomarkers related to neuronal damage and death (for example, neurofilament), and biomarkers related to muscle damage (for example, creatinine), it is surprising and of great clinical value that the blood uric acid level, which is routinely measured in clinical practice and is simple and non-invasive, can alone function as a biomarker for the above symptoms.

The blood uric acid level before administration of the medical agent of the present disclosure is preferably 4.3 mg/dL or higher, 4.4 mg/dL or higher, 4.5 mg/dL or higher, 4.6 mg/dL or higher, 4.7 mg/dL or higher, or 4.8 mg/dL or higher. Further, the blood uric acid level may be in a range that exceeds 7.0 mg/dL (the saturation solubility of uric acid in plasma), which can be diagnosed as hyperuricemia, for example, it may be in a range of 4.2 mg/dL to 7.0 mg/dL. The blood uric acid level may fluctuate throughout the day, but it can be measured at any time of the day. For example, it can be measured between meals, while fasting, or 2 to 3 hours after eating. It is preferable that the subject has never been administered 3-methyl-1-phenyl-2-pyrazolin-5-one or a physiologically acceptable salt thereof, or a hydrate or solvate thereof, before administration of the medical agent of the present disclosure, or that a long period, such as 6 months or more, or 1 year or more, has elapsed since the final administration of the compound. The blood uric acid level can be measured using the uricase-peroxidase method, which is commonly known in the technical field.

The subject is more preferably a subject for whom a difference (V2 - V1) between a blood uric acid level V1 before administration of the medical agent of the present disclosure and a blood uric acid level V2 two weeks or more from start of the administration of the medical agent of the present disclosure is 0 mg/dL or greater. Here, "0 mg/dL" can include values that round to 0.0 mg/dL when rounded to the second decimal place, that is, values in a range of -0.04 mg/dL to +0.04 mg/dL. The medical agent of the present disclosure can exert an excellent therapeutic effect or disease progression suppression effect by continuing to administer the medical agent to a subject whose blood uric acid level remains high or stable even after administration of the medical agent. The above V2 may be the blood uric acid level after 4 weeks or more, 6 weeks or more, 8 weeks or more, 10 weeks or more, 12 weeks or more, 14 weeks or more, 16 weeks or more, 18 weeks or more, 20 weeks or more, 22 weeks or more, or 24 weeks or more from the start of administration of the medical agent of the present disclosure. Further, the above V2 may be the blood uric acid level before 48 weeks have elapsed from the start of the administration of the medical agent of the present disclosure, or it may be the blood uric acid level measured between 2 weeks and 48 weeks from the start of the administration of the medical agent of the present disclosure. The blood uric acid level after the period from the start of the administration of the medical agent of the present disclosure also can be selected from the same range as the blood uric acid level before the start of the administration of the medical agent (for example, 4.2 mg/dL or higher, 4.3 mg/dL or higher, 4.4 mg/dL or higher, 4.5 mg/dL or higher, 4.6 mg/dL or higher, 4.7 mg/dL or higher, 4.8 mg/dL or higher, or the like).

In one embodiment, the subject can be a patient in need of treating or suppressing progression of ALS or a symptom resulting therefrom. The subject may have a Japanese ALS severity (hereinafter referred to as ALS severity) of grade 1, 2, 3, 4, or 5. The medical agent shows significant efficacy in subjects with an ALS severity of grade 1 or 2, but can also achieve significant efficacy in subjects with an ALS severity of grade 3, 4, or 5 (for example, grade 3 or 4), particularly in those who do not exhibit a decrease in blood uric acid level. Here, the ALS severity refers not only to the conditions defined bellow, which are published by the ALS Specific Disease Treatment Research Group of the Japanese Ministry of Health, Labor and Welfare, but also includes conditions equivalent to those definitions.
Grade 1: Generally able to do housework and work.
Grade 2: Difficulty with housework and work, but generally independent in daily life (taking care of oneself).
Grade 3: Unable to perform one or more of eating, excretion, or moving independently, and requires assistance in daily life.
Grade 4: Exhibiting respiratory distress, difficulty expectorating sputum, or swallowing disorder.
Grade 5: Requiring a tracheostomy, non-oral nutrient intake (tube feeding, total parenteral nutrition, and the like), or use of a respirator.

In one embodiment, the subject is a patient who requires assistance in daily life. The subject is preferably a patient who, for example, has difficulty or is unable to independently perform one or more (one, two, or three) of eating, excretion, and moving.

Further, the subject is preferably a patient who has one or more of the following characteristics 〈〈1〉〉 to 〈〈7〉〉.
((I)) Patient who meets the criteria of "ALS definite" or "ALS highly possible" according to the EL Escorial-Revised Airlie House diagnostic criteria;
((2)) Patient who meets the criteria of "ALS definite" according to the EL Escorial-Revised Airlie House diagnostic criteria;
((3)) Patient who scores 2 points or more with all items in ALSFRS-R;
((4)) Patient with an ALSFRS-R total score of 25 points or more, 30 points or more, 35 points or more, or 40 points or more;
((5)) Patient with %FVC of 70% or more or 80% or more;
((6)) Patient whose ALS severity is grade 3, 4, or 5, or who requires assistance in daily life (for example, having difficulty or being unable to independently perform one or more of eating, excretion, and moving);
〈〈7〉〉 Patient who meets 2, 3, 4, or 5 selected from 〈〈1〉〉 to 〈〈6〉〉 (for example, 〈〈3〉〉 and 〈〈4〉〉 ; 〈〈1〉〉 , 〈〈3〉〉 , and 〈〈4〉〉 ; 〈〈2〉〉 , 〈〈3〉〉 , and 〈〈4〉〉 ; 〈〈1〉〉 , 〈〈3〉〉 , 〈〈4〉〉 , 〈〈5〉〉 , and 〈〈6〉〉 ; or 〈〈2〉〉 , 〈〈3〉〉 , 〈〈4〉〉 , 〈〈5〉〉 , and 〈〈6〉〉 ).

The EL Escorial-Revised Airlie House diagnostic criteria are widely recognized diagnostic criteria worldwide, and are as shown in Fig. 1. Details of these diagnostic criteria are described in the Japan Society of Neurology Treatment Guidelines, ALS Treatment Guidelines 2002, III. Diagnosis/Differential Diagnosis, 3. Grading of Diagnostic Certainty.

The term "ALS definite" means that, when the body's motor control region is divided into 4 areas: the brainstem, cervical spinal cord, thoracic spinal cord, and lumbar-sacral spinal cord, clinical findings of upper and lower motor neuron damage are present in 3 or more of these areas.

The term "ALS highly possible" means that, when the body's motor control region is divided into 4 areas: the brainstem, cervical spinal cord, thoracic spinal cord, and lumbar-sacral spinal cord, clinical findings of upper and lower motor neuron damage are present in 2 of these areas.

The above ALSFRS-R is an abbreviation for The Revised ALS Functional Rating Scale, and refers to the following assessment scale aimed at understanding the daily life of an ALS patient. ALSFRS-R consists of 12 evaluation items in total regarding limb motor function impairment, bulbar function impairment, and respiratory function impairment. However, for eating/feeding, the items are selected and evaluated based on presence or absence of gastrostomy tube placement. (Neurology 53(4): 346-355, April 2001).

| Language | Dressing and personal movements |
|---|---|
| 4 : Speech is normal | 4 : Able to function normally |
| 3 : Speech is impaired | 3 : Able to do it completely alone with effort (or with low efficiency) |
| 2 : Meaning is understood after repeated listening | |
| | 2 : Occasional help or alternative methods needed |
| 1 : Use of non-vocal means of communication and speech | |
| | 1 : Needs help with daily activities |
| 0 : Loss of practical conversation | 0 : Totally dependent on others |

| Saliva secretion | Behavior in bed |
|---|---|
| 4 : Normal | 4 : Normal |
| 3 : Slight but clearly excessive saliva in the mouth (may drool at night) | 3 : Somewhat slow and clumsy, but does not need help |
| 2 : Moderate excess saliva (maybe a little drooling) | 2 : Can turn over and adjust bedding independently but with great difficulty |
| 1 : Noticeably excessive saliva (drooling) | 1 : Can start turning over but cannot complete the turn or adjust bedding independently |
| 0 : Profound drooling (constant need for tissues or handkerchiefs) | 0 : Unable to do anything by oneself |

| Swallowing | Walking |
|---|---|
| 4 : Normal eating habits | 4 : Normal |
| 3 : Early eating problems (occasionally choking on food) | 3 : Slightly difficult |
| | 2 : Assisted walking |
| 2 : Dietary consistency changes (cannot sustain eating continuously) | 1 : Unable to walk |
| | 0 : Unable to move legs |
| 1 : Requires supplemental tube feeding | |
| 0 : Totally parenteral or enteral feeding | |

| Writing | Stair climbing |
|---|---|
| 4 : Normal | 4 : Normal |
| 3Slow or scribbled (all words legible) | 3 : Slow |
| 2 : Some words illegible | 2 : Mild instability or fatigue |
| 1 : Can hold pen but cannot write | 1 : Needs assistance |
| 0 : Cannot hold pen | 0 : Unable to climb |
| Feeding behavior (evaluated based on either (1) or (2) depending on presence or absence of gastrostomy) | Breathing (Evaluated based on three items: dyspnea, orthopnea, and respiratory failure) |
| | (1) Dyspnea |
| (1) Use of eating utensils (without gastrostomy) | 4 : None |
| | 3 : Occurs during walking |
| 4 : Normal | 2 : Occurs during daily activities (eating, bathing, dressing) |
| 3 : Somewhat slow and clumsy, but does not require assistance | |
| | 1 : Occurs in sitting or lying positions |
| 2 : Can use a fork but not chopsticks | 0 : Extremely difficult, considering respiratory assistance |
| 1 : Needs someone to cut food but can eat with fork or spoon with effort | |
| | (2) Orthopnea |
| 0 : Needs to be fed by someone | 4 : None |
| (2) Finger movement (patients with gastrostomy) | 3 : Slight difficulty sleeping at night due to breathlessness |
| 4 : Normal | 2 : Requires pillows for support while sleeping |
| 3 : Awkward, but can do all hand movements | |
| 2 : Requires some assistance with buttons or zippers | 1 : Can only sleep in a sitting position |
| | 0 : Unable to sleep at all |
| 1 : Causes slight inconvenience to the caregiver | (3) Respiratory Failure |
| | 4 : None |
| 0 : Cannot do anything independently | 3 : Requires intermittent use of respiratory support device (BiPAP) |
| | 2 : Requires continuous use of respiratory support device (BiPAP) at night |
| | 1 : Requires use of respiratory support device (BiPAP) all day |
| | 0 : Requires mechanical ventilation through intubation or tracheostomy |

The above %FVC stands for percent-predicted forced vital capacity (%FVC), and is calculated according to the formula: (measured forced vital capacity (FVC) / predicted vital capacity) × 100. The predicted vital capacity is calculated based on gender, age, and height, and the formula for the calculation is "(27.63 - 0.112 × age) × height (cm)" for males, and "(21.78 - 0.101 × age) × height (cm)" for females. For ALS, a criterion for respiratory support is when %FVC is 50% or less (Japanese Society of Neurology Treatment Guidelines ALS Treatment Guidelines 2002, VIII. Respiratory Management and Nutritional Management).

For an administration method of the medical agent according to one embodiment of the present disclosure, for example, reference can be made to administration conditions using Edaravone injections. When used for treatment of ALS, the medical agent of the present disclosure can be administered, for example, using the administration method described in WO2020/091036 (or U.S. Patent Publication No. 2020/268712), or using the administration method using edaravone injections used in clinical practice. The administration method may be, for example, daily administration or intermittent administration. The intermittent administration is discontinuous administration, that is, for example, administration in which one unit (cycle constituting an administration period and a drug holiday period is repeated two or more times (for example, 2 to 24 times), preferably three or more times (for example, 3 to 18 times), and more preferably four or more times (for example, 4 to 12 times). When an administration period and a drug holiday period, as one unit, are repeated two or more times, the last period is a drug holiday period. In this case, the last drug holiday period may or may not be provided. That is, for example, when an administration period and a drug holiday period, as one unit, are repeated twice, it may be a case of "an administration period, a drug holiday period, an administration period, and a drug holiday period," or a case of "an administration period, a drug holiday period, and an administration period" without providing the last drug holiday period.

The drug holiday period refers to, for example, a period during which drug administration is not performed for several consecutive days (for example, 2 or 3 days) or more, and preferably a period during which drug administration is not performed for 7 or 14 consecutive days. The administration period is, for example, a 14-day period. In this case, during the administration period, administration may be performed continuously for 14 consecutive days, or administration may be performed for 10 days within the 14-day period. 10 days out of 14 days mean any 10 days out of 14 consecutive days. The 10 days in which administration is performed may be 10 consecutive days or 10 non-consecutive days separated by one or more periods (for example, periods of 1 to 4 days) in each of which administration is not performed. As the administration period, a preferable period can be selected while observing a condition of the patient. More specifically, for example, a method can be used that provides an initial 14-day administration period followed by an initial 14-day drug holiday period and then repeats a 10-day administration period within 14 days followed by a 14-day drug holiday period. There is no specific limitation on the number of repetitions of the 10-day administration period within 14 days and the 14-day drug holiday period, for example, it can be one or more times (for example, 1 to 23 times), preferably two or more times (for example, 2 to 17 times), and more preferably three or more times (for example, 3 to 11 times). In this repetition, the last drug holiday period may or may not be provided.

During an administration period, there is no limit to the number of doses per day, and a preferable number of doses can be selected while observing a condition of the patient. From a point or view of patient's burden, the number of doses is preferably 1, 2, or 3 times, more preferably 1 or 2 times, and even more preferably 1 time.

An administration route of the medical agent of the present disclosure is not particularly limited, and may be oral administration or parenteral administration. Further, bolus administration and sustained administration are possible. Sustained administration is preferable. In the case of sustained administration, intravenous administration by infusion, transdermal administration, oral administration using sublingual tablets, oral and rectal administration using sustained-release formulations, oral or gastric administration using suspensions, and the like can be used. Intravenous administration by infusion or oral or gastric administration using suspensions is preferable. In the case of administering a suspension to a subject multiple times, the administration may be gastric administration only, oral administration only, a combination of gastric administration and oral administration, or may be switched from oral administration to gastric administration or from gastric administration to oral administration. Generally, gastric administration means administering a medical agent directly into the stomach without passing through the mouth or esophagus. It is used as a measure to deal with cases where the patient is unable to ingest necessary nutrients through the mouth due to swallowing disorders and the like. Therefore, for example, in cases where more effective treatment is needed depending on the symptoms, switching from oral administration to gastric administration may be performed. Further, in the case of gastric administration, for example, administration via a tube (also referred to as enteral administration or enteral gastric administration) can be used. Specific examples include administration via a gastrostomy catheter or a nasogastric catheter, or a combination of both, or a switch from one to the other. In the case of performing bolus administration by injection or intravenous administration by infusion, it is preferable to use, for example, the injectable agents described in Japanese Patent Application Laid-Open Publication No. S63-132833 and Japanese Patent Application Laid-Open Publication No. 2011-62529 (or U.S. Patent Application Publication No. 2011/68037). In the case of performing oral or gastric administration, it is preferable to use, for example, suspensions such as those described in WO 2020/091036 (or U.S. Patent Application Publication No. 2020/0268712).

The medical agent of the present disclosure may be administered to a subject repeatedly every day or substantially every day during an administration period (also referred to as the daily administration). The daily dose of the active ingredient may be an effective amount, and can be selected appropriately depending on the age and condition (for example, severity of the disease) of the subject, the method of administration, and the like. For example, when administered intravenously to an adult, the daily dose of 3-methyl-1-phenyl-2-pyrazolin-5-one (the equivalent amount of 3-methyl-1-phenyl-2-pyrazolin-5-one when the active ingredient is a salt, hydrate, or solvate) is preferably about 15 mg to about 240 mg, more preferably about 30 mg to about 180 mg, even more preferably about 30 mg to about 60 mg or about 60 mg to about 120 mg, even more preferably about 60 mg or about 120 mg, and particularly preferably about 60 mg. Further, when administered orally or intragastricly to an adult, the dose of the active ingredient is preferably substantially equivalent pharmacokinetically to that administered intravenously. In one embodiment, it is a does at which change over time of 3-methyl-1-phenyl-2-pyrazolin-5-one unchanged concentration is found to be substantially equivalent. In such a case, the daily dose of 3-methyl-1-phenyl-2-pyrazolin-5-one has a lower limit of, for example, 60 mg or 90 mg and an upper limit of, for example, 400 mg, 140 mg, 120 mg, or 105 mg, and a range thereof is, for example, 60 mg to 400 mg, preferably 90 mg to 140 mg, more preferably 90 mg to 120 mg, even more preferably 90 mg to 105 mg, particularly preferably 90 mg, 100 mg, 105 mg, or 120 mg, of which 100 mg or 105 mg is preferred, and most preferably 105 mg.

Edaravone injection (Japanese brand name "Radicut," registered trademark) used in clinical practice as an ALS therapeutic agent, is set to have a dose of 60 mg of edaravone per administration. When the medical agent of the present disclosure is a medical agent for oral administration or intragastric administration, in order to achieve an effect equivalent to when the edaravone injection (60 mg of edaravone per administration) is intravenously administered, it is preferable to set a dose per administration to 90 mg to 105 mg converted to 3-methyl-1-phenyl-2-pyrazolin-5-one, and more specifically, it is preferable to set the dose to 105 mg converted to 3-methyl-1-phenyl-2-pyrazolin-5-one.

### 2. Method

A method of the present disclosure is for treating or suppressing progression ofat least one symptom selected from a group consisting of ALS and symptoms resulting from ALS in a subject in need of treating or suppressing progression of the symptom. The method includes a step of administering to the subject 3-methyl-1-phenyl-2-pyrazolin-5-one or a physiologically acceptable salt thereof, or a hydrate or solvate thereof. The subject has a blood uric acid level of 4.2 mg/dL or higher before the administration. Structural components of this method (symptoms, subjects, active ingredients, doses, administration methods, and the like) can adopt the same components as described in the above 1.

### 3. Compound

A compound of the present disclosure is 3-methyl-1-phenyl-2-pyrazolin-5-one or a physiologically acceptable salt thereof, or a hydrate or solvate thereof, for use in treating or suppressing progression of at least one symptom selected from a group consisting of ALS and symptoms resulting from ALS in a subject. The subject has a blood uric acid level of 4.2 mg/dL or higher before administration of the compound. Structural components of this compound (symptoms, subjects, active ingredients, doses, administration methods, and the like) can adopt the same components as described in the above 1.

### 4. Use

A use of the present disclosure is a use of 3-methyl-1-phenyl-2-pyrazolin-5-one or a physiologically acceptable salt thereof, or a hydrate or solvate thereof, for manufacturing a medical agent for treating or suppressing progression of at least one symptom selected from a group consisting of ALS and symptoms resulting from ALS in a subject. The subject has a blood uric acid level of 4.2 mg/dL or higher before administration of the medical agent. Structural components of this use (symptoms, subjects, active ingredients, doses, administration methods, and the like) can adopt the same components as described in the above 1.

### Examples

In the following, the embodiments of the present disclosure are described in more detail. However, the present disclosure is not limited to these embodiments.

### 1. Method

### 1-1. Trial Details

Clinical trials using edaravone injections include two-group phase 3 trials. In the first phase 3 trials [MCI186-16 trial (hereinafter referred to as the 16 trial) and MCI186-17 trial (hereinafter referred to as the 17 trial)], it was shown that edaravone was involved in suppressing decline in ALS function compared to placebo, as evaluated by an ALSFRS-R total score. A subgroup showing a significant therapeutic effect of Edaravone has been identified by the 16 trial, and an inclusion criterion for this subgroup is also used in designing a second major phase 3 trial, that is, a 19 trial (MCI186-19). The 19 trial showed a remarkable effect of edaravone, specifically, the least squares (LS) mean ± standard deviation of the change in ALSFRS-R from baseline to 24 weeks was -7.50±0.66 for placebo, whereas it was -5.01±0.64 for edaravone (p = 0.0013).

The 16 trial was a randomized, double-blind, parallel-group, placebo-controlled trial. Details of the trial are as described in NCT00330681, Reference 1 (Amyotroph Lateral Scler Frontotemporal Degener 2014;15 (7-8):610-617) and Reference 2 (Amyotroph Lateral Scler Frontotemporal Degener 2017;18 (suppl 1): 11-19). The 17 trial was an extension trial from the 16 trial. Details of the trial are as described in NCT00424463.

After a 12-week observation period, patients whose ALSFRS-R total score decreased by 1 to 4 points during that period were considered eligible for the trial. In the 16 trial, eligible patients were randomly divided into an edaravone treatment group and a placebo administration group, and patients in each group received administration for 24 weeks (cycles 1 to 6). After completing the 24-week double-blind portion of the 16 trial, patients who received edaravone (E) were randomly assigned to the 17 trial, and during a 24-week double-blind extension period (cycles 7 to 12), edaravone treatment (EE group) or placebo administration (EP group) was performed. On the other hand, all patients from the placebo (P) administration group received edaravone treatment (PE group). All patients received open-label edaravone treatment for the next 12 weeks (cycles 13 to 15).

Edaravone was administered at a dose of 60 mg by intravenous infusion over 60 minutes, once daily. The infusion was given for 14 days in the first treatment cycle, and for 10 days out of the 14-day treatment period in all subsequent cycles. A 14-day drug holiday period was provided after each treatment cycle.

The primary efficacy endpoint was the change in the ALSFRS-R total score from baseline to the end of 24 weeks. The second endpoint included changes in (1) predicted forced vital capacity (%FVC), (2) Modified Norris Scale scores (limb, bulbar, and total), (3) ALSAQ-40 total score, (4) ALS severity, and (5) grip strength and pinch strength. The safety evaluation included reports of adverse events and specific clinical laboratory test items.

The MCI186-19 trial (hereinafter referred to as the 19 trial) was a randomized, double-blind, parallel-group, placebo-controlled trial. Details of the trial are as described in NCT01492686, Reference 3 (Lancet Neurol 2017;16 (7):505-512) and Reference 4 (Amyotroph Lateral Scler Frontotemporal Degener 2017;18 (suppl 1):55-63).

Similar to the 16 trial, after a 12-week observation period, patients in the 19 trial were randomly divided into an edaravone treatment group and a placebo administration group, and patients in each group received administration for 24 weeks (cycles 1 to 6). In the 19 trial, after the double-blind treatment period, all patients received open-label edaravone treatment for an additional 24 weeks (cycles 7 to 12). Therefore, the 19 trial included an EE group and a PE group.

### 1-2. Patients

Main selection criteria for the 16 and 17 trials were as follows: age 17 to 75 years; diagnosed as "ALS definite," "ALS highly possible," or "ALS highly possible with positive test findings" based on the EL Escorial-Revised Airlie House diagnostic criteria; predicted forced vital capacity (%FVC) ≥70% (predicted); disease duration ≤3 years from first symptom (any ALS symptom); and ALS severity of grade 1 or 2.

Main selection criteria for the 19 trial were the same as the 16 trial, except that the patients had to have an ALSFRS-R item score ≥2 for all 12 items of the ALSFRS-R; be diagnosed as "ALS definite" or "ALS highly possible" based on the EL Escorial-Revised Airlie House diagnostic criteria; have %FVC ≥80% (predicted); and have a disease duration ≤2 years.

### 1-3. Method for measuring uric acid levels

The blood uric acid levels in the 16 trial, 17 trial, and 19 trial were measured values obtained using the uricase-peroxidase method.

### 1-4. Post-hoc statistical analysis 1

In the present analysis, efficacy was analyzed for placebo patients who had completed the 24-week double-blind period (cycles 1 to 6) and had at least one data point in cycle 7. To estimate the percent change in ALSFRS-R slope for transition from placebo administration (baseline to 24 weeks) to edaravone administration (24 weeks 24 to 48 weeks), a random coefficient model was applied to all available ALSFRS-R score data during this period.

A 25% improvement group meets both of the following criteria:
(1) ALSFRS-R slope % change ≥ +25% (this change is defined as a clinically significant improvement (Reference 5: Amyotroph Lateral Scler 2010; 11 (1-2): 178-180)) and (2) ALSFRS-R total score at the start of cycle 7 (24 weeks) > 24 points (half of the 48-point ALSFRS-R total score) (this helps avoid floor effects related to the ALSFRS-R total score that could lead to false positives in the analysis).

These criteria were used to define a 25% improvement group and a non-25% improvement group. Using a mixed model for repeated measures, the changes in uric acid levels from the start of cycle 7 (24 weeks) to cycle 12 (48 weeks) were summarized for the 25% improvement group and the non-25% improvement group. The correlation between the change in uric acid levels from 24 weeks to 48 weeks and the ALSFRS-R slope percent change was descriptively summarized using the Wilcoxon test.

### 1-5. Post-hoc statistical analysis 2

In order to characterize the uric acid level and ALSFRS-R total score profiles at cycle 12, efficacy was analyzed in randomized ALS patients at the start of cycle 7 and randomized ALS patients who received the medical agent at least once after the start of cycle 7.

Descriptive statistics (number of subjects, mean, standard deviation, minimum, maximum) were used to summarize the changes in uric acid levels (mg/dL) and ALSFRS-R total scores from baseline to cycle 12 for the entire population and for the Edaravone treatment group as a subgroup defined by the median baseline uric acid level (4.8 mg/dL). The median baseline uric acid level for all patients (4.8 mg/dL) was derived using the entire edaravone population, including the EE, PE, and EP groups in the 16 trial, the 17 trial, and the 19 trial.

The least squares (LS) mean difference of the EE and EP treatment groups compared to the PE treatment group was estimated using a mixed-effects model for repeated measures (MMRM). By using the MMRM, the LS mean, standard error, 95% confidence interval (CI), and p-value for each group are calculated. The MMRM includes treatment group, time point, interaction between treatment group and time point, age (<65 years or ≥65 years), initial symptoms (bulbar or limb symptoms), ALS diagnostic criteria (ALS definite, ALS highly possible, ALS highly possible with positive test findings, ALS possible), change in ALSFRS-R total score during the 12-week observation period (-1/-2 or -3/-4), and use of riluzole (Yes or No) as fixed effects, and includes baseline of the ALSFRS-R total score or uric acid baseline (mg/dL) of the median subgroup as covariates. A compound symmetric covariance matrix was applied to model covariance of within-patient scores, and the Kenward-Roger approximation was applied to estimate a degrees of freedom of the criteria. The statistical significance level was set at a two-sided level of 0.05.

In a sensitivity analysis, propensity score matching cohort evaluation was conducted to compare the baseline changes in ALSFRS-R total score at 48 weeks between the EE group from the 16 trial, the 17 trial, and the 19 trial, and a matching cohort from the Pooled Resource Open-Access ALS Clinical Trials Database (hereinafter PRO-ACT) database. The EE group and PRO-ACT placebo group were matched 1:1 using a caliper width of 0.2 of the logit of the propensity score. The propensity score was developed using baseline confounding factors and ALS progression variables (gender, body weight, age, duration of ALS disease, ALSFRS-R total score baseline, uric acid baseline) of the EE group. A balance after the matching was evaluated using a standardized mean difference of less than 0.1 (Reference 6: J Clin Epidemiol 2001;54 (4): 387-398). The propensity score was added as an explanatory variable for the MMRM analysis. Further, a subgroup analysis of uric acid levels was performed using MMRM analysis within the matching cohort, and the change from baseline in the ALSFRS-R total score at 48 weeks was compared between the EE group and the PRO-ACT placebo group.

### 2. Results of Post-hoc Statistical Analysis 1

### 2-1. Baseline Characteristics

The present analysis included a total of 146 patients, including 88 patients from the placebo-edaravone treatment groups of the 16 trial and the 17 trial, and 58 patients from the placebo-edaravone treatment group of the 19 trial. Composition and baseline characteristics of the patients were similar, except for the ALS diagnostic criteria and ALS severity. In particular, at the start of cycle 7, 34% (30 out of 88 patients) of the patients in the 16 trial and the 17 trial had ALS severity of grade 3 to grade 5, while 53% (31 out of 58 patients) of patients in the 19 trial had ALS severity of grade 3 to grade 5.

### 2-2. Post-hoc analysis

### 2-2-1. Percent change in ALSFRS-R slope derived from random coefficient model, improved with edaravone treatment compared to placebo

The proportion of patients showing improvement in ALSFRS-R slope upon transition from placebo to edaravone treatment was 43.2% (95% CI: 35.0% to 51.6%) for the entire placebo-edaravone treatment population. The proportion of patients with at least a 25% improvement in ALSFRS-R slope, having a cutoff of 24 points, was 24.0% (95% CI: 17.3% to 31.7%) for the entire placebo-edaravone treatment population. When analyzed by ALS severity, the proportion of patients showing improvement in ALSFRS-R slope was 64.3% (95% CI: 48.0% to 78.5%) for severity grade 3 and 66.7% (95% CI: 41.0% to 86.7%) for severity grade 4. Further, the proportion of patients with at least a 25% improvement in ALSFRS-R slope, having a cutoff of 24 points, was 35.7% (95% CI: 21.6% to 52.0%) for severity grade 3 and 33.3% (95% CI: 13.3% to 59.0%) for severity grade 4.

### 2-2-2. Baseline Characteristics of the 25% Improvement Group and Non-25% Improvement Group

Baseline factors were generally similar between the 25% improvement group and non-25% improvement group, except for mean weight and ALS severity (Table 1). The mean weight in the 25% improvement group was 56.9 kg, which was lower than 59.8 kg in the non-25% improvement group. Regarding baseline ALS severity, the proportion of patients with severity grade 2 was 82.9% in the 25% improvement group, which was higher than 59.5% in the non-25% improvement group. Additionally, at the start of cycle 7, the proportion of patients with severity grades 3 and 4 was 60% (21 out of 35 patients) in the 25% improvement group, which was higher than 35% (39 out of 111 patients) in the non-25% improvement group.

**[Table 1]**

| **Table 1 Composition and clinical characteristics of 25% improvement group and non-25% improvement group** | | |
|---|---|---|
| **Variables** | **25% improvement group (n=35)** | **non-25% improvement group (n=111)** |
| **Gender, n (%)** | | |
| Male | 23 (65.7) | 70 (63.1) |
| Female | 12 (34.3) | 41 (36.9) |
| **Age, yr** | 60.5 (10.1) | 57.2 (10.3) |
| **Baseline weight of cycle 1, kg** | 56.9 (8.2) | 59.8 (10.4) |

| **ALSdiagnosis, n (%)** | | |
|---|---|---|
| ALSdefinitely | 8 (22.9) | 31 (27.9) |
| ALShighly possible | 20 (57.1) | 63 (56.8) |
| ALSwith positive test findings | 7 (20.0) | 16 (14.4) |
| ALSpossible | 0 (0.0) | 1 (0.9) |

| **Cycle 1, baseline** | | |
|---|---|---|
| **ALSseverity, n(%)** | | |
| Severity grade 1 | 6 (17.1) | 45 (40.5) |
| Severity grade 2 | 29 (82.9) | 66 (59.5) |

| **At start of cycle 7 (24 weeks)** | | |
|---|---|---|
| **ALSseverity, n(%)** | | |
| Severity grade 1 | 2 (5.7) | 12 (10.8) |
| Severity grade 2 | 12 (34.3) | 58 (52.3) |
| Severity grade 3 | 15 (42.9) | 27 (24.3) |
| Severity grade 4 | 6 (17.1) | 12 (10.8) |
| Severity grade 5 | 0 (0.0) | 1 (0.9) |
| **Average disease duration, yr** | 1.2 (0.5) | 1.2 (0.6) |

| **Early symptom, n (%)** | | |
|---|---|---|
| Bulbar onset | 7 (20.0) | 21 (18.9) |
| Limb onset | 28 (80.0) | 90 (81.1) |
| **ALSFRS-R total score at cycle 1 baseline (BL) and at start of cycle 7 (24 weeks)** | BL: 40.6 (1.88) | BL: 41.7 (2.76) |
| | Cycle 7: 33.3 (4.96) | Cycle 7: 36.1 (6.87) |
| **%FVC at cycle 1 baseline (BL) and at start of cycle 7 (24 weeks)** | BL: 94.4 (13.93) | BL: 98.4 (15.96) |
| | Cycle 7: 78.1 (20.10) | Cycle 7: 82.7 (23.24) |
| **Uric acid level at cycle 1 baseline (BL) and at start of cycle 7 (24 weeks)** | BL: 4.80 (1.334) | BL: 4.79 (1.351) |
| | Cycle 7: 4.58 (1.405) | Cycle 7: 4.61 (1.275) |
| **Serum creatinine at cycle 1 baseline (BL) and at start of cycle 7 (24 weeks)** | BL: 0.53 (0.131) | BL: 0.57 (0.149) |
| | Cycle 7: 0.47 (0.141) | Cycle 7: 0.52 (0.154) |

### 2-2-3. Change in uric acid levels from the start of cycle 7 to the final administration of cycle 12

Table 2 shows the uric acid levels and changes thereof. Regarding the change in uric acid level from the start of cycle 7 to the final administration of cycle 7 (2 weeks after starting edaravone administration), the change in the 25% improvement group (mean: 0.05 mg/dL, LS mean: -0.16 mg/dL) was smaller than the change in the non-25% improvement group (mean: -0.14 mg/dL, LS mean: -0.37 mg/dL), with the LS mean difference between the two groups being 0.21 mg/dL (p = 0.0726). Further, regarding the change in uric acid level from the start of cycle 7 to the final administration of cycle 12, the change in the 25% improvement group (mean: -0.04 mg/dL, LS mean: -0.28 mg/dL) was smaller than the change in the non-25% improvement group (mean: - 0.22 mg/dL, LS mean: -0.56 mg/dL), with the LS mean difference between the two groups being 0.28 mg/dL (p = 0.0252). Further, similar results for ALS severity of grades 3 to 5 are shown in Table 3.

**[Table 2]**

| **Table 2. Uric acid levels (mg/dL) in 25% improvement group and non-25% improvement group** | | |
|---|---|---|
| **Variables** | **25% improvement group** | **non-25% improvement group** |
| Baseline at start of cycle 7 (week 24) | | |
| n | 35 | 111 |
| Mean (SD) | 4.58 (1.41) | 4.61 (1.27) |

| Final administration of Cycle 7 | | |
|---|---|---|
| n | 35 | 110 |
| Mean (SD) | 4.63 (1.45) | 4.46 (1.29) |
| Change from start of Cycle 7 to final administration of Cycle 7 | | |
| | 35 | 110 |
| n | 0.05 (0.49) | -0.14 (0.52) |
| Mean (SD) | -0.16 (0.15) | -0.37 (0.12) |
| LSmean* (SE) | | |
| LSMD* (SE) between 25% improvement group and non-25% improvement group | 0.21 (0.12) | |
| | p=0.0726 | |
| Final administration of Cycle 12 | | |
| n | 32 | 84 |
| Mean (SD) | 4.51 (1.43) | 4.48 (1.29) |

| Change from start of Cycle 7 to final administration of Cycle 12 | | |
|---|---|---|
| n | 32 | 84 |
| Mean (SD) | -0.04 (0.68) | -0.22 (0.81) |
| LSmean* (SE) | -0.28 (0.15) | -0.56 (0.12) |
| LSMD* (SE) between 25% improvement group and non-25% improvement group | 0.28 (0.12) | |
| | p=0.0252 | |

| | | |
|---|---|---|
| *: LS mean = least squares mean, LSMD = least squares mean difference | | |

**[Table 3]**

| **Table 3. Uric acid levels (mg/dL) in 25% improvement and non-25% improvement groups with ALS severity grade 3 to 5** | | |
|---|---|---|
| **Variables** | **25% improvement group** | **non-25% improvement group** |
| Baseline at start of cycle 7 (week 24) | | |
| n | 21 | 40 |
| Mean (SD) | 4.60 (1.35) | 4.53 (1.13) |

| Final administration of Cycle 7 | | |
|---|---|---|
| n | 21 | 39 |
| Mean (SD) | 4.65 (1.49) | 4.31 (1.10) |

| Change from start of Cycle 7 to final administration of Cycle 12 | | |
|---|---|---|
| n | 21 | 39 |
| Mean (SD) | 0.05 (0.47) | -0.22 (0.60) |
| LSmean* (SE) | -0.37 (0.24) | -0.61 (0.21) |
| LSMD* (SE) between 25% improvement group and non-25% improvement group | 0.24 (0.18) | |
| | p=0.1701 | |

| Final administration of Cycle 12 | | |
|---|---|---|
| n | 18 | 24 |
| Mean (SD) | 4.61 (1.48) | 4.18 (1.00) |

| Change from start of Cycle 7 to final administration of Cycle 12 | | |
|---|---|---|
| n | 18 | 24 |
| Mean (SD) | 0.04 (0.60) | -0.40 (1.02) |
| LSmean* (SE) | -0.42 (0.24) | -0.98 (0.22) |
| LSMD* (SE) between 25% improvement group and non-25% improvement group | 0.55 (0.19) | |
| | p=0.0044 | |

| | | |
|---|---|---|
| *: LS mean = least squares mean, LSMD = least squares mean difference | | |

2-2-4. Correlation between uric acid levels and the percentage change in ALSFRS-R slope Patients (n=41) whose uric acid levels increased from the start of cycle 7 to the final administration of cycle 12 were analyzed in comparison to patients (n=105) whose uric acid levels remained unchanged or decreased. There was no correlation between the change in uric acid level and percent change in ALSFRS-R slope (r = 0.06, p = 0.4868). However, the median percent change in ALSFRS-R slope was 14% in the group with a positive change in uric acid level, which was significantly higher than the group with a negative change in uric acid level (-38%, p = 0.0433, Table 4).

**[Table 4]**

| **Table 4. Percentage change in ALSFRS-R slope due to change in uric acid level (mg/dL) (>0 or ≦0) from start of cycle 7 to last administration of cycle 12** | | |
|---|---|---|
| **Variables** | **Change in uric acid level >0 (n=41)** | **Change in uric acid level ≦0 (n=105)** |
| Mean (SD) | -64 (199) | -201 (664) |
| Median | 14 | -38 |
| (Q1, Q3) | (-86,43) | (-130, -21) |
| (Min, Max) | (-819, 87) | (-5102 104) |
| P-value in Wilcoxon test | 0.0433 | |

### 2-2-5. Summary

In the present analysis, rates of improvement in ALSFRS-R slope during placebo administration and subsequent edaravone administration were compared, and background characteristics of the improvement group were evaluated. In doing so, it was found that the improvement group included ALS patients with severity grades 3 or 4. Further, when investigating background factors contributing to the improvement in ALSFRS-R slope using LASSO machine learning, a change in uric acid level was identified as a candidate factor. Further investigation revealed an association between changes in uric acid levels and changes in improvement of the ALSFRS-R slope, suggesting an association between suppression of decrease in uric acid levels under edaravone administration and suppression of decrease in ALSFRS-R. Further, in the improvement group of ALS patients with ALS severity of grade 3 or 4, suppression of decrease in uric acid levels was observed. In addition, even in severe cases, the effectiveness of edaravone can be expected when uric acid levels are still preserved under edaravone administration.

Since the patients included in the present analysis were assigned to the placebo group and administered placebo for 6 months, the severity of ALS at the start of edaravone administration was expected to be higher than at the start of placebo administration. However, in the placebo-edaravone group of ALS patients in the present analysis, despite being assigned to the placebo group and administered placebo for 6 months, it was shown that after starting edaravone administration, 43% of the patients had improvement in ALSFRS-R slope. Further, 24% of the patients had ALSFRS-R total scores exceeding 24 points at the start of cycle 7, and had an improvement of 25% or more in ALSFRS-R slope, indicating a clinically significant change.

In the clinical development of the present medical agent, the efficacy of the present medical agent for ALS severity of grade 3 was examined in a very small number of cases in an exploratory trial (18 trial, Reference 7: Amyotroph Lateral Scler Frontotemporal Degeneration 2017;18 (suppl 1):40-48), and no statistically significant difference was observed compared to placebo regarding the change in ALSFRS-R total score. However, in the present analysis, a 25% improvement in ALSFRS-R slope of edaravone compared to placebo was observed in about one-third (21/60 cases) of patients with ALS severity of grade 3 or 4 at the start of cycle 7, and the 25% improvement group included 21 patients (60% of 35 cases) with ALS severity of grade 3 or 4 at the start of cycle 7. From this data, it was found that a certain number of patients with ALS severity of grade 3 or 4 responded to edaravone. Therefore, in addition to the benefits of edaravone already demonstrated in the 19 trial for patients with ALS severity of grades 1 and 2, the present analysis suggests that edaravone can also provide benefits for patients with higher severity grades.

The present analysis showed that the increase in uric acid after the start of edaravone treatment is associated with a positive change in ALSFRS-R slope. In addition, patients with an increase in uric acid level after the start of edaravone treatment showed less decrease in ALSFRS-R total score compared to patients with a decrease in uric acid level.

### 3. Results of Post-hoc Statistical Analysis 2

### 3-1. Baseline Characteristics

The 304 patients in the combined data sets of the 16 trial, the 17 trial, and the 19 trial include 113 EE group patients, 45 EP group patients, and 146 PE group patients. For the propensity score matching cohort, there are 80 patients from the EE group and 80 matched patients from the PRO-ACT database.

Composition and baseline characteristics of the patients were highly similar between the EE group, the EP group, and the PE group in the 16 trial, the 17 trial, and the 19 trial, except for gender and age. Regarding gender, the proportion of males was 73% in the EP group, which was higher than 56% in the EE group and 64% in the PE group. The mean age was 55 years in the EP group, which was younger than 60 years in the EE group and 58 years in the PE group.

Patients were analyzed based on whether their baseline uric acid level was above or below the mean uric acid level (4.8 mg/dL). When comparing patients with high or low baseline uric acid levels in the EE group, the EP group, and the PE group, there were some differences in the composition and baseline characteristics of the patients. The proportion of females was 48% to 61% in a low uric acid level subgroup, compared to 13% to 26% in a high uric acid level subgroup. The mean weight was 54 kg to 60 kg in the low uric acid level subgroup, compared to 62 kg to 63 kg in the high uric acid level subgroup. The proportion of bulbar-onset patients was 20% to 29% in the low uric acid level subgroup, compared to 11% to 18% in the high uric acid level subgroup.

In the matched cohort, the composition and baseline characteristics of the patients were equivalent between the EE group and the matched PRO-ACT placebo group, confirming the propensity score matching (Table 5). Specifically, in both groups, the mean ALSFRS-R total score was about 41 points, and the mean uric acid level was 4.9 mg/dL.

**[Table 5]**

| **T able 5. Baseline composition and clinical characteristics in propensity matched cohort** | | |
|---|---|---|
| | **Edaravone-Edaravone (EE) group (n=80)** | **Placebo PRO-ACT matching group (n=80)** |
| ***Overall*** | | |
| **Gender, n (%)** | | |
| Male | 47 (58.8) | 49 (61.3) |
| Female | 33 (41.3) | 31 (38.8) |
| **Weight (SD), kg** | 61.2 (11.1) | 62.7 (10.9) |
| **Mean age (SD), yr** | 59.1 (10.3) | 58.9 (10.9) |
| **Mean disease duration (SD), yr** | 1.3 (0.6) | 1.3 (0.4) |
| **MeanALSFRS-Rscore (SD)** | | |
| Cycle 1 baseline | 41.0 (3.45) | 40.9 (4.11) |
| **Mean uric acid level (mg/dL) (SD)** | | |
| Cycle 1 baseline | 4.9 (1.20) | 4.9 (1.26) |

| ***Subgroup with uric acid level baseline <4.8 mg*/*dL*** | | |
|---|---|---|
| | **EE (n=38)** | **placebo (n=37)** |
| **Gender, n (%)** | | |
| Male | 16 (42.1) | 15 (40.5) |
| Female | 22 (57.9) | 22 (59.5) |
| **Weight (SD), kg** | 57.3 (9.8) | 58.4 (9.2) |
| **Mean age (SD), yr** | 59.2 (10.3) | 60.4 (9.4) |
| **Mean disease duration (SD), yr** | 1.2 (0.4) | 1.3 (0.5) |
| **MeanALSFRS-Rscore (SD)** | | |
| Cycle 1 baseline | 41.1 (2.56) | 40.6 (4.61) |
| **Mean uric acid level (mg/dL) (SD)** | | |
| **Cycle 1 baseline** | **3.9 (0.70)** | **3.9 (0.70)** |

| ***Subgroup with uric acid level baseline ≧4.8 mg*/*dL*** | | |
|---|---|---|
| | **EE (n=42)** | **placebo (n=43)** |
| **Gender, n (%)** | | |
| Male | 31 (73.8) | 34 (79.1) |
| Female | 11 (26.2) | 9 (20.9) |
| **Weight (SD), kg** | 64.6 (11.2) | 66.4 (11.1) |
| **Mean age (SD), yr** | 59.0 (10.4) | 57.6 (12.0) |
| **Mean disease duration (SD), yr** | 1.3 (0.7) | 1.3 (0.5) |
| **Mean ALSFRS-Rscore (SD)** | | |
| Cycle 1 baseline | 40.9 (4.12) | 41.1 (3.67) |
| **Mean uric acid level (mg/dL) (SD)** | | |
| Cycle 1 baseline | 5.7 (0.89) | 5.9 (0.85) |

### 3-2. Descriptive Summary of Uric Acid Levels from Baseline to Cycle 12

The baseline uric acid levels were not balanced among the three edaravone groups (Table 6). In particular, the high baseline uric acid level was 5.1 mg/dL in the EP group, compared to 4.7 mg/dL in the EE group and 4.8 mg/dL in the PE group. The change from baseline to cycle 12 was -0.25 mg/dL in the EE group and -0.22 mg/dL in the EP group, which were slightly higher than -0.37 mg/dL in the PE group. The EE group and the EP group showed slightly smaller reductions compared to the PE group.

In the low uric acid level baseline subgroup, the baseline uric acid levels were generally similar, with the PE group having the smallest uric acid level baseline (3.6 mg/dL) among the three treatment groups (Table 10 and Fig. 6). The changes in uric acid levels from baseline to cycle 12 were similar across the EE group, the EP group, and the PE group (-0.1 mg/dL to a maximum of +0.15 mg/dL).

In the high uric acid level baseline subgroup, the uric acid baseline levels were similar across the treatment groups (Table 6). The change in uric acid level from baseline to cycle 12 was -0.4 mg/dL in the EE group, -0.48 mg/dL in the EP group, which were smaller than -0.68 mg/dL in the PE group. The EE group and the EP group showed smaller decreases in uric acid levels compared to the PE group.

**[Table 6]**

| **Table 6. Uric acid levels (mg/dL) from baseline to cycle 12 in population of 16 trial, 17 trial, and 19 trial** | | | |
|---|---|---|---|
| | **Edaravone-Edaravone (EE)** | **Edaravone-Placebo (EP)** | **Placebo-Edaravone (PE)** |
| ***Overall*** | | | |
| **Baseline (BL)** | | | |
| n | 113 | 45 | 146 |
| Mean (SD) | 4.7 (1.27) | 5.1 (1.29) | 4.8 (1.34) |
| (Min, Max) | (1.8, 8.6) | (2.3, 8.4) | (0.7, 8.9) |
| **Cycle 12** | | | |
| n | 98 | 41 | 116 |
| Mean (SD) | 4.4 (1.36) | 4.9 (1.20) | 4.5 (1.32) |
| (Min, Max) | (1.7, 9.3) | (2.5, 8.1) | (0.7, 8.5) |
| **Change from BL to cycle 12** | | | |
| n | 98 | 41 | 116 |
| Mean (SD) | -0.25 (0.86) | -0.22 (0.77) | -0.37 (0.86) |
| (Min, Max) | (-3.2, 1.7) | (-1.8, 1.1) | (-2.4, 2.0) |

| ***Subgroup with uric acid level baseline <4.8 mg*/*dL*** | | | |
|---|---|---|---|
| **Baseline (BL)** | | | |
| n | 59 | 17 | 70 |
| Mean (SD) | 3.8 (0.70) | 3.9 (0.65) | 3.6 (0.72) |
| (Min, Max) | (1.8, 4.7) | (2.3, 4.7) | (0.7, 4.7) |
| **Cycle 12** | | | |
| n | 52 | 17 | 53 |
| Mean (SD) | 3.6 (0.85) | 4.1 (0.93) | 3.6 (0.95) |
| (Min, Max) | (1.7, 5.4) | (2.5, 5.5) | (0.7, 5.6) |
| **Change from BL to cycle 12** | | | |
| n | 52 | 17 | 53 |
| Mean (SD) | -0.10 (0.69) | 0.15 (0.64) | 0.00 (0.71) |
| (Min, Max) | (-1.8, 1.7) | (-0.8, 1.1) | (-1.5, 2.0) |

| ***Subgroup with uric acid level baseline ≧4.8 mg*/*dL*** | | | |
|---|---|---|---|
| **Baseline (BL)** | | | |
| n | 54 | 28 | 76 |
| Mean (SD) | 5.8 (0.86) | 5.9 (0.99) | 5.9 (0.80) |
| (Min, Max) | (4.8, 8.6) | (4.8, 8.4) | (4.8, 8.9) |
| **Cycle 12** | | | |
| n | 46 | 24 | 63 |
| Mean (SD) | 5.4 (1.21) | 5.5 (1.04) | 5.2 (1.17) |
| (Min, Max) | (2.9,9.3) | (3.7, 8.1) | (3.1, 8.5) |
| **Change from BL to cycle 12** | | | |
| n | 46 | 24 | 63 |
| Mean (SD) | -0.41 (1.01) | -0.48 (0.77) | -0.68 (0.86) |
| (Min, Max) | (-3.2, 1.5) | (-1.8, 1.1) | (-2.4, 0.8) |

### 3-3. Descriptive statistical summary of ALSFRS-R total score from baseline to cycle 12

In the three edaravone groups, the baseline ALSFRS-R total scores were similar, at about 41 points (Table 7). The change in ALSFRS-R total score from baseline to cycle 12 was smallest (a change of -8.1 points) in the EE group among the three edaravone groups.

In the low uric acid level baseline subgroup and high uric acid level baseline subgroup, the baseline ALSFRS-R total scores were similar across the three edaravone groups, at about 41 points (Table 7). The change in ALSFRS-R total score from baseline to cycle 12 was -8.7 points (low uric acid baseline subgroup) and -7.4 points (high uric acid baseline subgroup) in the EE group, showing the smallest decrease among the three treatment groups.

### 3-4. Descriptive statistical summary of change in ALSFRS-R total score from baseline to cycle 12

In the overall trial population, the least squares mean difference (LSMD) in the change in ALSFRS-R total score from baseline to cycle 12 was 2.75 between the EE group and the PE group, favoring the EE group (95% CI [1.55, 3.94], p < 0.0001, Table 7).

In the low uric acid level baseline subgroup, the LSMD in the change in ALSFRS-R total score from baseline to cycle 12 was 1.53 between the EE group and the PE group, favoring the EE group, although this was not statistically significant (95% CI [-0.23, 3.29], p = 0.0882, Table 7).

In the high uric acid level baseline subgroup, the LSMD in the change in ALSFRS-R total score from baseline to cycle 12 was 3.88 between the EE group and the PE group, favoring the EE group (95% CI [2.19, 5.56], p < 0.0001, Table 7).

Thus, the therapeutic effect of edaravone was more evident in the high uric acid level baseline subgroup than in the low uric acid level baseline subgroup. The EP group showed a favorable trend compared to the PE group, with an estimated score being higher than 1.0 points. However, due to the small sample size of the PE group, the results were not statistically significant in the overall population and the two uric acid level subgroups.

**[Table 7]**

| **Table 7. Change from baseline to cycle 12 in ALSFRS-R total score in population of 16 trial, 17 trial, and 19 trial** | | | |
|---|---|---|---|
| | Edaravone-Edaravone (EE) | Edaravone-Placebo (EP) | Placebo-Edaravone (PE) |
| ***Overall*** | | | |
| **Baseline (BL)** | | | |
| n | 113 | 44 | 146 |
| Mean (SD) | 41.3 (3.21) | 40.9 (3.10) | 41.5 (2.62) |
| (Min, Max) | (29, 47) | (34, 47) | (32, 47) |
| **Cycle 12** | | | |
| n | 95 | 40 | 112 |
| Mean (SD) | 33.3 (7.66) | 31.3 (7.74) | 31.2 (8.64) |
| (Min, Max) | (11, 46) | (18, 48) | (4, 47) |
| **Change from BL to cycle 12** | | | |
| n | 95 | 40 | 112 |
| Mean (SD) | -8.1 (5.97) | -9.6 (6.88) | -10.3 (7.70) |
| (Min, Max) | (-27, 2) | (-25, 1) | (-35, 0) |
| **LS Mean* (SE)** | -7.2 (1.17) | -8.7 (1.28) | -9.9 (1.12) |
| **LSMD* vs PE group (SE)** | 2.75 (0.61) | 1.20 (0.83) | N/A |
| **[95%CI]*** | [1.55,3.94] | [-0.42,2.82] | |
| **p-value** | <0.0001 | 0.1469 | |

| ***Subgroup with uric acid level baseline <4.8 mg*/*dL*** | | | |
|---|---|---|---|
| **Baseline (BL)** | | | |
| n | 59 | 17 | 70 |
| Mean (SD) | 41.4 (2.55) | 41.2 (2.80) | 41.3 (2.31) |
| (Min, Max) | (35, 45) | (35, 47) | (36, 46) |
| **Cycle 12** | | | |
| n | 50 | 17 | 51 |
| Mean (SD) | 32.6 (8.48) | 31.5 (7.66) | 31.6 (7.99) |
| (Min, Max) | (11, 45) | (18, 48) | (10, 44) |
| **Change from BL to cycle 12** | | | |
| n | 50 | 17 | 51 |
| Mean (SD) | -8.7 (6.95) | -9.7(7.17) | -9.7 (7.30) |
| (Min, Max) | (-27, 1) | (-22, 1) | (-27, 0) |
| **LS Mean* (SE)** | -7.0 (1.38) | -7.8 (1.78) | -8.5 (1.37) |
| **LSMD* vs PE group** | 1.53 (0.89) | 0.68 (1.33) | N/A |
| **(SE)** | [-0.23, 3.29] | [-1.94,3.29] | |
| **[95%CI]*** | 0.0882 | 0.6119 | |
| **p-value** | | | |

| ***Subgroup with uric acid level baseline ≧4.8 mg*/*dL*** | | | |
|---|---|---|---|
| **Baseline (BL)** | | | |
| n | 54 | 27 | 76 |
| Mean (SD) | 41.1 (3.82) | 40.7 (3.32) | 41.6 (2.88) |
| (Min, Max) | (29, 47) | (34, 46) | (32, 47) |
| **Cycle 12** | | | |
| n | 45 | 23 | 61 |
| Mean (SD) | 34.0 (6.65) | 31.1 (7.97) | 30.8 (9.20) |
| (Min, Max) | (22, 46) | (18, 46) | (4, 47) |
| **Change from BL to cycle 12** | | | |
| n | 45 | 23 | 61 |
| Mean (SD) | -7.4 (4.63) | -9.5 (6.82) | -10.9 (8.03) |
| (Min, Max) | (-19, 2) | (-25, 0) | (-35, 0) |
| **LS Mean* (SE)** | -8.1 (0.91) | -10.3 (1.05) | -12.0 (0.77) |
| **LSMD* vs PE group (SE)** | 3.88 (0.86) | 1.69 (1.07) | N/A |
| **[95%CI]*** | [2.19,5.56] | [-0.41, 3.79] | |
| **p-value** | <0.0001 | 0.1136 | |

| | | | |
|---|---|---|---|
| *: LS mean = least squares mean, LSMD = least squares mean difference, 95%CI = 95% confidence interval | | | |

### 3-5. Propensity score matching cohort evaluation (sensitivity evaluation) for comparing change in ALSFRS-R total score from baseline to cycle 12

In the propensity score matching cohort analysis, the baseline ALSFRS-R scores were equivalent at around 41 points in both the low uric acid level baseline subgroup and the high uric acid level baseline subgroup (Table 8). The change in ALSFRS-R score from baseline to cycle 12 was -8.7±0.53 in the EE group, and -10.9±0.55 in the PRO-ACT placebo group (Table 8). The LSMD between the groups was 2.21±0.77 (95% CI [0.71, 3.72], p=0.0041), favoring the EE group (Table 8). Further, in the scope of the present analysis, the treatment difference between the EE group and the matching PRO-ACT placebo group was larger in the high uric acid level baseline subgroup than in the low uric acid level baseline subgroup (Table 8). These results are consistent with the comparison between the EE group and the PE group, and the results of both analyses indicate a greater therapeutic effect of edaravone in patients with high uric acid level baseline.

**[Table 8]**

| **Table 8. ALSFRS-R total score from baseline to cycle 12 in propensity matching cohort** | | |
|---|---|---|
| | **Edaravone-Edaravone (EE) group** | **Placebo-PRO-ACT matching group** |
| ***Overall*** | | |
| **ALSFRS-R total score** | | |
| **Baseline (BL)** | | |
| n | 80 | 80 |
| Mean (SD) | 41.0 (3.45) | 40.9 (4.11) |
| (Min, Max) | (29,47) | (29, 47) |
| **ALSFRS-R total score at cycle 12** | | |
| n | 69 | 55 |
| Mean (SD) | 32.8 (7.79) | 31.2 (8.35) |
| (Min, Max) | (11, 46) | (14, 46) |
| **Change from baseline to cycle 12** | | |
| n | 69 | 55 |
| Mean (SD) | -8.2 (6.16) | -9.9 (6.15) |
| (Min, Max) | (-27, 2) | (-27, -1) |
| **LS Mean* (SE)** | -8.7 (0.53) | -10.9 (0.55) |
| **LSMD* vs placebo PRO-ACT (SE)** | 2.21 (0.77) | N/A |
| **[95%CI]*** | [0.71,3.72] | |
| **p-value** | 0.0041 | |

| ***Subgroup with uric acid level baseline <4.8 mg*/*dL*** | | |
|---|---|---|
| **ALSFRS-R total score** | | |
| **Baseline (BL)** n | 38 | 37 |
| Mean (SD) | 41.1 (2.56) | 40.6 (4.61) |
| (Min, Max) | (35, 45) | (29, 47) |
| **ALSFRS-R total score at cycle 12** | | |
| n | 31 | 25 |
| Mean (SD) | 31.3 (8.79) | 30.6 (7.79) |
| (Min, Max) | (11, 43) | (16,42) |
| **Change from baseline to cycle 12** | | |
| n | 31 | 25 |
| Mean (SD) | -9.6 (7.52) | -10.0 (5.63) |
| (Min, Max) | (-27, 1) | (-27, -3) |
| **LS Mean* (SE)** | -10.3 (0.74) | -10.7 (0.79) |
| **LSMD* vs placebo PRO-ACT (SE)** | 0.45 (1.09) | N/A |
| **[95%CI]*** | [-1.69,2.60] | |
| **p-value** | 0.6761 | |

| ***Subgroup with uric acid level baseline ≧4.8 mg*/*dL*** | | |
|---|---|---|
| **ALSFRS-R total score** | | |
| **Baseline (BL)** | | |
| n | 42 | 43 |
| Mean (SD) | 40.9 (4.12) | 41.1 (3.67) |
| (Min, Max) | (29,47) | (31, 47) |
| **ALSFRS-R total score at cycle 12** | | |
| n | 38 | 30 |
| Mean (SD) | 34.0 (6.75) | 31.7 (8.89) |
| (Min, Max) | (22, 46) | (14, 46) |
| **Change from baseline to cycle 12** | | |
| n | 38 | 30 |
| Mean (SD) | -7.1 (4.58) | -9.7 (6.64) |
| (Min, Max) | (-16, 2) | (-25, -1) |
| **LS Mean* (SE)** | -7.3 (0.73) | -11.0 (0.76) |
| **LSMD* vs placebo PRO-ACT (SE)** | 3.63 (1.06) | N/A |
| **[95%CI]*** | [1.55, 5.71] | |
| **p-value** | 0.0007 | |

| | | |
|---|---|---|
| *: LS mean = least squares mean, LSMD = least squares mean difference, 95%CI = 95% confidence interval | | |

### 3-6. Descriptive Summary of Uric Acid Levels from Baseline to Cycle 6

For uric acid levels and their changes from baseline, additional analyses were conducted, limited to patients in the placebo-controlled double-blind period (cycles 1-6), to confirm the pharmacological effect of uric acid on edaravone treatment. Similar results were obtained, with the edaravone group at cycle 6 showing a smaller decrease in uric acid levels compared to the placebo group. However, the difference was smaller than that observed from baseline to cycle 12.

### 3-7. Summary

Initially, the relationship between baseline uric acid levels and changes in ALSFRS-R total score due to edaravone treatment was evaluated using an integrated edaravone dataset. This analysis showed that the EE group was associated with both a decrease in uric acid levels and a decrease in ALSFRS-R scores from baseline compared to the PE group. The EP group showed a small decrease in uric acid levels from baseline compared to the PE group, although there was no statistically significant difference in the decrease in ALSFRS-R scores. Further, compared to ALS patients with low baseline uric acid levels, ALS patients with high baseline uric acid levels showed suppression of decrease in both uric acid levels and ALSFRS-R scores in the EE group.

Secondly, since the control group in the above analyses was the PE group, in order to compare with a placebo group, a matching cohort of the EE group was created using PROACT placebo data, and the robustness of the above results was evaluated by comparing the EE group and the PROACT placebo group. Comparing with the PROACT placebo group, the EE group showed a decrease in ALSFRS-R, with more significant therapeutic effects observed in ALS patients with higher uric acid levels compared to those with lower uric acid levels.

Due to the very small number of cases in the PE group, no statistically significant difference was observed between the EP group and the PE group. However, the decrease in ALSFRS-R total score from baseline was smaller in the EP group compared to the PE group. There is a possibility showing that progression of ALS is suppressed by protecting nerve cells with early administration of edaravone. This data suggests that early edaravone administration may be beneficial in delaying disease progression.

Based on the results of the present analysis, edaravone treatment was shown to delay the decrease in both uric acid levels and ALSFRS-R total scores more effectively in the high uric acid level baseline subgroup patients compared to the low uric acid level baseline subgroup patients. It is considered that the effect of edaravone on ALSFRS-R by suppressing the decrease in uric acid levels was more prominently observed in patients with higher uric acid levels.

On the other hand, in the subgroup with low uric acid levels, there was no statistically significant difference between the EE group and the PE group. In this subgroup, no further decrease in uric acid levels was observed because the uric acid levels were already low at baseline. Since uric acid, which has various antioxidant effects, was already low at baseline, it is possible that this subgroup did not experience as much delay in the decrease in ALSFRS-R as the high uric acid subgroup. However, in the subgroup with low uric acid levels, the EE group tended to show improvement in ALSFRS-R compared to the PE group and the PROACT placebo group. Therefore, it is considered that there is still significance in administering edaravone even in patients with low uric acid levels.

### 4. Post-hoc statistical analysis of MT-1186-A01 trial

The MT-1186-A01 trial is an international collaborative Phase III trial using edaravone oral suspension (Edaravone 105mg) identified by NCT04165824 or EudraCT Number 2019-002108-41. For the MT-1186-A01 trial population, a subgroup analysis similar to the post-hoc statistical analysis 2 was conducted. The least squares mean difference (LSMD) in the change from baseline to 24 weeks in ALSFRS-R total score was 1.37 between the baseline uric acid levels & 4.2 mg/dL and < 4.2 mg/dL, favoring baseline uric acid levels ≥ 4.2 mg/dL (95%CI [0.06, 2.68], p=0.0411, Table 9). The uric acid levels in the present trial were measured using the uricase-peroxidase method.

**[Table 9]**

| **Table 9. Change from baseline to 24 weeks in ALSFRS-R total score in MT-1186-A01 trial population** | | |
|---|---|---|
| | Oral edaravone Baseline uric acid level ≧4.2 mg/dL | Oral edaravone Baseline uric acid level <4.2 mg/dL |
| **Baseline (BL)** | | |
| n | 145 | 40 |
| Mean (SD) | 40.1 (4.59) | 39.6 (4.27) |
| (Min, Max) | (22, 48) | (27, 47) |
| **Change from BL to 24 weeks** | | |
| n | 132 | 36 |
| Mean (SD) | -4.9 (4.71) | -6.4 (5.29) |
| (Min, Max) | (-20,5) | (-20,0) |
| **LS Mean* (SE)** | -5.0(0.31) | -6.4 (0.59) |
| **LSMD* vs baseline uric acid level <4.2 mg/dL group (SE)** | | |
| | 1.37 (0.67) | N/A |
| **[95%CI]*** | [0.06, 2.68] | |
| **p-value** | 0.0411 | |

| | | |
|---|---|---|
| *: LS mean = least squares mean, LSMD = least squares mean difference, 95%CI = 95% confidence interval | | |

The MT-1186-J03 trial identified by NCT04493281 was conducted as a Phase I, open-label, single-dose, crossover trial in healthy subjects, and examined bioequivalence between edaravone oral suspension and edaravone injection (already approved drug). As a result, in a pharmacokinetic profile of administration of an oral suspension containing 105 mg of edaravone, equivalence to edaravone 60 mg / 60 minute intravenous administration was confirmed based on AUC (area under the curve). Based on the above, the baseline uric acid levels found from the results of the 16 trial, the 17 trial, and the 19 trial using edaravone injections and from the results of the MT-1186-A01 trial using edaravone oral suspensions are considered to be applicable regardless of the administration route of edaravone.

## Claims

1. A medical agent for treating or suppressing progression of at least one symptom selected from a group consisting of amyotrophic lateral sclerosis and symptoms resulting from amyotrophic lateral sclerosis in a subject, comprising:
3-methyl-1-phenyl-2-pyrazolin-5-one or a physiologically acceptable salt thereof, or a hydrate or solvate thereof, wherein
a blood uric acid level of the subject before administration of the medical agent is 4.2 mg/dL or higher.

2. The medical agent according to claim 1, wherein the blood uric acid level is 4.5 mg/dL or higher.

3. The medical agent according to claim 1, wherein the blood uric acid level is 4.8 mg/dL or higher.

4. The medical agent according to any one of claims 1 to 3, wherein the subject is a subject for whom a difference (V2 - V1) between a blood uric acid level V1 before administration of the medical agent and a blood uric acid level V2 two weeks or more from start of the administration of the medical agent is greater than 0 mg/dL.

5. The medical agent according to any one of claims 1 to 4, wherein the administration of the medical agent is intermittent administration or daily administration.

6. The medical agent according to any one of claims 1 to 5, wherein
an administration route of the medical agent is oral administration or gastric administration, and
a dose per administration of the medical agent is 90 mg to 120 mg converted to 3-methyl-1-phenyl-2-pyrazolin-5-one.

7. The medical agent according to any one of claims 1 to 6, wherein
an administration route of the medical agent is oral administration or gastric administration, and
a dose per administration of the medical agent is 90 mg to 105 mg converted to 3-methyl-1-phenyl-2-pyrazolin-5-one.

8. The medical agent according to any one of claims 1 to 7, wherein
the administration of the medical agent is daily administration,
an administration route of the medical agent is oral administration or gastric administration, the medical agent is administered once a day, and
a dose per administration of the medical agent is 105 mg converted to 3-methyl-1-phenyl-2-pyrazolin-5-one.

9. The medical agent according to any one of claims 1 to 7, wherein
the administration of the medical agent is intermittent administration, and
the medical agent is administered in a regimen that repeats a 14-day administration period and a 14-day drug holiday period, or, after an initial 14-day administration period and a subsequent initial 14-day drug holiday period, repeats a 10-day administration period out of 14 days followed by a 14-day drug holiday period.

10. The medical agent according to any one of claims 1 to 7, wherein
the administration of the medical agent is intermittent administration, and
the medical agent is administered in a regimen that, after an initial 14-day administration period and a subsequent initial 14-day drug holiday period, repeats a 10-day administration period out of 14 days followed by a 14-day drug holiday period.

11. The medical agent according to any one of claims 1 to 10, wherein the subject has an amyotrophic lateral sclerosis (ALS) severity of grade 3, 4, or 5.

12. The medical agent according to any one of claims 1 to 11, wherein the symptom resulting from amyotrophic lateral sclerosis include decreased respiratory function, speech impairment, dysphagia, and limb motor dysfunction.
